# EUROPÄISCHE PATENTANMELDUNG

(11) **EP 4 257 097 A1**
(43) Veröffentlichungstag der Anmeldung: **11.10.2023**
(21) Anmeldenummer: 22166476.6
(22) Anmeldetag: 04.04.2022
(51) Int. Cl.: A61F 5/44

(54) **BLICKDICHTER HYGIENEBEUTEL**

(71) Anmelder: Human Nutrition GmbH, 55234 Erbes-Büdesheim (DE)
(72) Erfinder: Wendel, Hermann J., 55288 Armsheim (DE)
(74) Vertreter: Zellentin & Partner mbB Patentanwälte

(57) **Zusammenfassung**

Hygienebeutel (1) zur Aufnahme von Körperflüssigkeit, umfassend einen Flüssigkeitssammelbehälter (2), der durch eine vorderseitige Folie und eine rückseitige Folie, die miteinander verbunden sind, gebildet wird, wobei die vorderseitige Folie einen deckenden Druck (4) aufweist, durch den die Körperflüssigkeit nicht sichtbar ist.

## Beschreibung

Die vorliegende Erfindung betrifft einen Hygienebeutel zur Aufnahme von Körperflüssigkeiten, insbesondere einen Urinbeutel, in dem der Inhalt vor Blicken geschützt ist.

Hygienebeutel zum Sammeln von Körperflüssigkeiten, insbesondere Urinbeutel, sind in der Pflege bekannt und werden täglich millionenfach verwendet. Urinbeutel dienen dazu, eine Körperflüssigkeit wie Urin, der mittels Katheter aus der Blase abgeleitet wird, aufzufangen und sicher zu verwahren. Ist ein vorgegebener Füllstand erreicht, erfolgt eine Entleerung, die möglichst sicher und vollständig gewährleistet sein soll. Urinbeutel können auch als Ersatz der Blase bezeichnet werden. Sie werden sowohl bei Männern als auch bei Frauen angewendet. Die Verbindung zur Blase und zum Blaseninneren wird mittels eines Katheters hergestellt. Üblicherweise wird der Katheter in die Urethra geschoben und mit Hilfe eines wassergefüllten Ballons in der Blase festgelegt. Der abfließende Urin wird in dem Beutel gesammelt.

Ein solcher Urinbeutel umfasst in der Regel einen Flüssigkeitssammelbehälter (kurz Behälter), der typischerweise aus zwei miteinander verschweißten Folien besteht, wobei die vordere Folie durchsichtig ist und die hintere aus einem undurchsichtigen Material besteht, und einem Anschlussschlauch, der mit dem Katheter verbunden wird. Das Volumen des Behälters beträgt normalerweise 0,5 bis 4 I. Häufig ist auch ein Abfluss zum Entleeren vorgesehen, zumeist ein Schlauch mit einer Ritsch-Ratsch-Klemme. Eine häufige Gestaltung sieht vor, dass die Vorderseite des Beutels mit einer Skala versehen ist, um die gesammelte Urinmenge abschätzen zu können. Unzählige Varianten und Ausführungsformen von Urinbeuteln bzw. Hygienebeuteln sind möglich und in Gebrauch. Diese sind dem Fachmann bekannt und bedürfen hier keiner näheren Beschreibung. Beispielhaft sei auf DE 202005001925 T2, EP 847742 B1 und EP 748620 B1 verwiesen.

Patienten empfinden die durchsichtigen Beutel jedoch häufig als unangenehm. Man sieht oft in Krankenhäusern, dass Betroffene, die ihre Urinbeutel mittragen bzw. am Rollstuhl befestigen, den Urinbeutel in einer Tragetasche verstauen. Dies ist nicht einem leichteren Tragen geschuldet, sondern mehr der Tatsache, dass Betroffene sich durch den transparenten Beutel in der Intimsphäre verletzt fühlen, den Urin nicht öffentlich ausstellen möchten.

Es werden bereits Möglichkeiten angeboten, wie die Intimsphäre besser geschützt werden kann. So sind Taschen bekannt, die aus Baumwolle gefertigt sind und um den Beutel gelegt werden können. Diese haben eine Öffnung an der Oberseite, aus der der Schlauch, der die Verbindung mit dem Katheter darstellt, geführt werden kann und manchmal eine Öffnung an der Unterseite zum Entleeren des Beutels. Auch Taschen aus anderen Materialien sind bekannt und werden für denselben Zweck genutzt. Beispielhaft für diese Vorschläge sind DE 20 2013 002 466 U1, DE 20 2013 000 927 U1, DE 20 2012 001 065 U1,DE 29 17 583 C2, DE 20 2005 016 479 U1, DE 296 14 690 U1, DE 202 07 270 U1, DE 82 159 93 U1, CH 711 038 A2 und US 5,135,519 A.

Alle diese Taschen haben den großen Nachteil, dass die Qualität des Urins nicht beurteilt werden kann. Auch die Füllmenge kann nicht abgelesen werden, da die Taschen die auf den Beutel aufgedruckte Skala verdecken. Für das Entleeren sind viele der Taschen umständlich, da der Beutel zum Entleeren häufig erst aus der Tasche ausgepackt werden muss, danach entleert und dann wieder eingepackt werden muss. Das kann dazu führen, dass die Tasche durch den Urin verschmutzt wird. Außerdem sind solche Taschen teuer.

Auch in Bezug auf Stomabeutel sind bereits Sichtschutzeinrichtungen vorgeschlagen worden. Die US 4,533,355 A schlägt eine Hose vor, die den Stomabeutel in einer Tasche oder gedoppelten Vorderpartie aufnimmt und zusätzlich eine oberhalb der Aufnahme befestigte Abdeckung vorsieht. Gemäß US 5,799,180 soll eine Abdeckung auf die Vorderseite des Stomabeutels aufgeklebt werden. Auch diese Vorschläge erlauben bei Übertragung auf Urinbeutel nicht die gewünschte einfache Kontrolle von Füllstand und Urinqualität.

Ein weiterer Vorschlag gemäß EP 2 853 246 B1 sieht vor, zusätzlich zu den beiden für den Beutel notwendigen Folien eine dritte undurchsichtige Folie am oberen Rand des Beutels zu befestigen und bei Bedarf hochzuklappen.

Alle diese beispielhaft genannten Vorschläge haben den Nachteil, dass weiteres Material und Arbeitszeit benötigt wird und sich die Entstehungskosten des Beutels erhöhen. Beim Beispiel einer zusätzlichen Folie würden sich die Herstellungskosten um ca. 40% erhöhen, außerdem würde sie die Umwelt um 50 % mehr belasten. Bei einem zusätzlichen Beutel würden sich die Kosten sogar mehr als verdoppeln.

Da die Krankenversicherungen, die den Beutel zahlen, aber über Ausschreibungen, Festpreis oder über Vertragspreise die Preise festlegen, ist eine Erhöhung der Herstellungskosten am Markt nicht durchsetzbar.

Es besteht daher weiter die Aufgabe einen Sichtschutz für Hygienebeutel, besonders Urinbeutel, bereitzustellen, der die Intimsphäre schützt, der günstig zu fertigen ist, kein zusätzliches Material benötigt und einfach in der Handhabung ist.

Überraschend wurde nun gefunden, dass diese Aufgabe durch eine undurchsichtige Bedruckung der Vorderseite eines Hygienebeutels erfüllt wird.

Die Erfindung löst daher die obigen Aufgaben durch einen Hygienebeutel zur Aufnahme von Körperflüssigkeiten, vorzugsweise einen Urinbeutel, umfassend einen Flüssigkeitssammelbehälter, der von einer vorderseitigen Folie und einer rückseitigen Folie, die miteinander verbunden sind, gebildet wird, wobei die vorderseitige Folie einen deckenden Druck aufweist. Der Druck verhindert, dass die Körperflüssigkeit in dem Sammelbehälter sichtbar ist.

Der erfindungsgemäße Hygienebeutel umfasst einen Flüssigkeitssammelbehälter, der wie an sich bekannt durch miteinander verbundenen Folien gebildet wird. Es können zwei separate Folienstücke verwendet werden oder ein aufeinandergefaltetes Folienstück. Die Verbindung der vorderseitigen Folie mit der rückseitigen Folie erfolgt in der Regel durch Verschweißen, beispielsweise thermisch oder mittels Ultraschall, an den Rändern. Aber auch andere Verfahren wie Kleben sind denkbar. Üblicherweise werden die notwendigen Anschlüsse beim Verbinden direkt mit angebracht. Insoweit entspricht der erfindungsgemäße Hygienebeutel den bekannten Einwegbeuteln.

Der erfindungsgemäße Hygienebeutel unterscheidet sich von den oben bereits beschriebenen, bekannten Beuteln dadurch, dass die Vorderseite, die normalerweise durchsichtig ist, deckend bedruckt wird. Der Druck kann einfarbig, z.B. blau, grün oder weiß, sein, aber auch mehrfarbig, z.B. kariert, gestreift oder anderweitig grafisch gestaltet.

Es kann eine Anzeige für den Füllstand in Form einer Ausnehmung von dem Druck vorgesehen sein. An der vorgesehenen Stelle wird kein Druck aufgebracht. Es entsteht ein Fenster, das verschiedene Formen haben kann, z.B. rund, insbesondere kreisförmig oder oval, oder eckig, insbesondere rautenförmig oder quadratisch. Andere Formen sind ebenfalls denkbar, wie rechteckig, trapezförmig, sechseckig usw. Das durch die Ausnehmung gebildete Fenster erstreckt sich in einem Bereich der vorderseitigen Folie, an dem der Blick auf die Flüssigkeit zum Ablesen des Füllstands zweckmäßig ist. Mit anderen Worten, das Fenster soll eine vertikale Ausdehnung haben, die eine Beobachtung der zunehmenden Füllung des Beutels mit Flüssigkeit erlaubt.

In der einfachsten Ausgestaltung kann das Fenster ein vertikaler Streifen mit Skala sein. Dies ist jedoch weniger bevorzugt, da der Hygienebeutel bei dieser Gestaltung unmittelbar "nach einem Hygienebeutel" aussieht. Daher wird vorzugsweise bei einem Fenster in Form eines einfachen, vertikalen Streifens zumindest ein Druck vorgesehen, der die Anzeige optisch in den Hintergrund treten lässt. Denkbar sind u.a. Streifenmuster, beispielsweise in vielen Farben, oder Bilder, bei denen der durchsichtige Streifen in das Motiv integriert ist.

In einer bevorzugten Variante werden mehrere kleine Ausnehmungen in dem Druck vorgesehen, die vorzugsweise in einer Linie übereinander angeordnet sind. Geeignet sind z.B. 3 bis 20, vorzugsweise 5 bis 15, besonders bevorzugt 6 bis 12, Ausnehmungen, bevorzugt an einem Rand der vorderseitigen Folie. Die Ausnehmungen sind zweckmäßig gleich groß. Ebenso können aber auch Ausnehmungen mit vertikal stetig ansteigender Größe oder abwechselnd größere und kleinere Ausnehmungen vorliegen. Es reicht aus, wenn die Ausnehmungen im Vergleich zur Folienoberfläche klein sind, z.B. jede Ausnehmung eine Fläche im Bereich von 0,05 bis 0,25 % der Gesamtfläche der vorderseitigen Folie hat, vorzugsweise von 0,1 bis 0,2 %. In absoluten Zahlen sind Flächen von 0,1 bis 0,5 cm², vorzugsweise bis 0,2 cm² oder bis 0,15 cm², geeignet. Durch eine solche Reihe von kleinen Ausnehmungen kann der Füllstand besonders einfach und schnell beurteilt werden.

In einer weiteren, bevorzugten Ausführungsform ist die Ausnehmung ein Streifen, z.B. in Form der Kontur eines Blattes, einer Blume oder eines sonstigen Gegenstandes. Es wird also ein nicht bedruckter Streifen vorgesehen, dessen Form dem Umriss, ggfs. inklusive einiger Innenlinien, eines Gegenstandes entspricht. Beim Füllen des Beutels wird der mit der Kontur angedeutete Gegenstand, d.h. die Blume oder das Blatt etc., z.B. durch Urin gelb, gefärbt und so kann der Füllstand abgelesen werden. Andere Formen sind vorstellbar und ebenfalls eingeschlossen. Auch hier ist die vertikale Ausdehnung so angepasst, dass zumindest die Flüssigkeit in dem Bereich, in welchem der Füllstand einen Leerungsbedarf anzeigt, sichtbar ist.

Die Rückseite des Beutels, die bisher oft undurchsichtig ist, kann erfindungsgemäß ebenfalls aus einer durchsichtigen Folie, vorzugsweise mit einem weiteren Druck, bestehen. Dabei kann die Rückseite so gestaltet werden, dass bei einem deckenden Druck die benötigten Anzeigen, wie die genaue Füllmenge oder die Anzeige der Kleinstmenge beim Bedrucken ausgenommen werden. Weiter kann am unteren Ende erfindungsgemäß eine, z.B. sichelförmige, Ausnehmung in dem Druck gestaltet werden, um die Qualität der gesammelten Körperflüssigkeit, z.B. des Urins, zu beurteilen. Außerdem ist es möglich, dass die rückseitige Folie durchsichtig bleibt und nur die benötigten Anzeigen aufgedruckt sind.

Üblicherweise befindet sich auf der Vorderseite des Hygienebeutels am oberen Ende der Einlaufschlauch und am unteren Ende der Ablassschlauch bzw. Ablasshahn. Um die Erkennbarkeit als Hygienebeutel weiter zu erschweren, wird vorzugsweise erfindungsgemäß der Ablassschlauch bzw. Ablasshahn auf die Rückseite des Beutels verlegt. Der Einlaufschlauch bleibt auf der Vorderseite. Es ist auch eine umgekehrte Anordnung möglich, bis hin zu beiden Anschlüssen auf einer Seite, vorzugsweise der Rückseite.

Die Erfindung soll anhand der beigefügten Figuren näher erläutert werden, ohne jedoch auf die speziell beschriebenen Ausführungsformen beschränkt zu sein. Die Erfindung bezieht sich auch auf sämtliche Kombinationen von bevorzugten Ausgestaltungen, soweit diese sich nicht gegenseitig ausschließen.

### Es zeigen:

Figur 1 einen Urinbeutel mit separater Folie als Sichtschutz wie er heute gemäß dem Stand der Technik verwendet wird
Figur 2 zeigt die Vorderseite eines erfindungsgemäßen Urinbeutels mit gedrucktem Sichtschutz und Ausnehmung in Form eines Kleeblatts als Füllstandsanzeige
Figur 3 zeigt die Vorderseite eines erfindungsgemäßen Urinbeutels mit gedrucktem Sichtschutz und Ausnehmung in Form einer Reihe von 9 Kreisen als Füllstandsanzeige
Figur 4 zeigt die Rückseite eines erfindungsgemäßen Beutels mit gedrucktem Sichtschutz, Ausnehmungen für zwei Füllstandsanzeigen und am unteren Ende eine sichelförmige Ausnehmung, um die Qualität des Urins zu beurteilen.

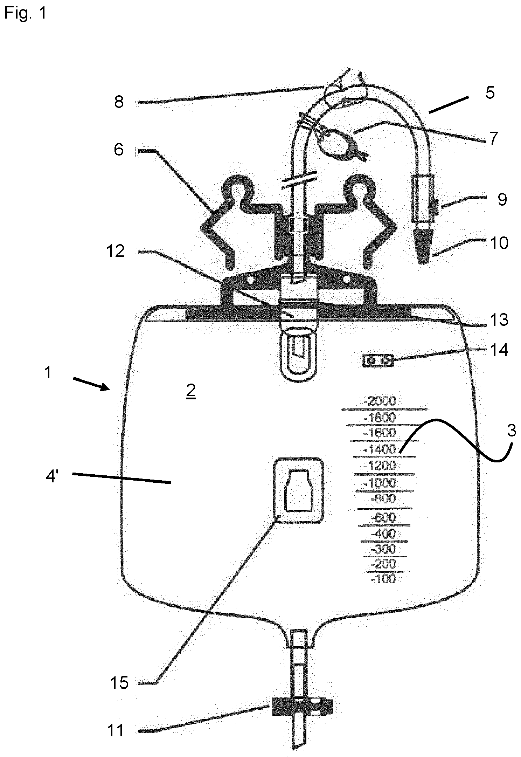

In Figur 1 ist ein Urinbeutel 1 gezeigt, wie er vom Grundaufbau her im Stand der Technik bekannt ist. Der Beutel 1 umfasst den Flüssigkeitssammelbehälter 2, der von Folienstücken gebildet wird, die miteinander verschweißt sind. Auf dem Behälter 2 ist eine Skalierung 3 aufgedruckt, mit deren Hilfe der Füllstand beurteilt werden kann. Die Vorderseite des Behälters 2 ist ansonsten mit einem Sichtschutz in Form einer separaten Folie 4' abgedeckt. Oben sieht man den Schlauch 5, welcher den Anschluss zum Katheter (nicht gezeigt) herstellt. Außerdem verfügt der Beutel 1 über eine Aufhängung 6, eine Schlauchfixierklemme 7, eine Ritsch-Ratsch-Klemme 8, eine Probenentnahmestelle 9 und eine Schutzkappe 10. Unten befindet sich der Ablasshahn 11, über den der Beutel 1, genauer der Behälter 2, entleert werden kann.

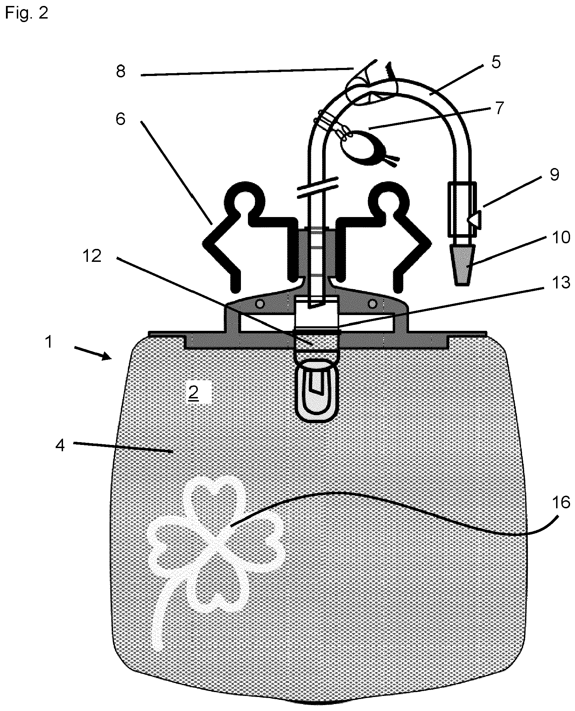

In Figur 2 ist ein erfindungsgemäßer Urinbeutel 1 gezeigt, bei dem die vorderseitige Folie mit einem deckenden Druck 4 versehen ist, in der Figur als Punktschraffur angedeutet. Der Druck 4 auf der vorderseitigen Folie weist außerdem eine Ausnehmung in Form einer Kleeblattkontur auf, die ein Fenster 16 bildet. Durch das Fenster 16 kann der Füllstand in dem Flüssigkeitssammelbehälter 2 kontrolliert werden. Der Urinbeutel 1 in Figur 2 hat einen hinten am Flüssigkeitssammelbehälter 2 angebrachten Ablasshahn 11, so dass dieser von vorne nicht zu sehen ist.

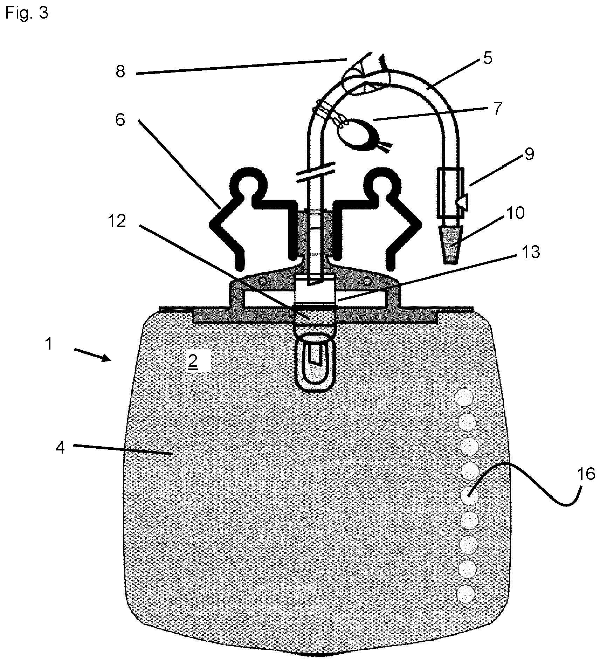

Figur 3 zeigt eine Ausführungsform eines erfindungsgemäßen Urinbeutels 1 mit einem Fenster 16 in Form einer Vielzahl kreisförmiger Ausnehmungen, die übereinander angeordnet sind. Analog zu der Skalierung beim Beutel 1 des Standes der Technik in Figur 1 kann der Füllstand hier genauer beurteilt werden.

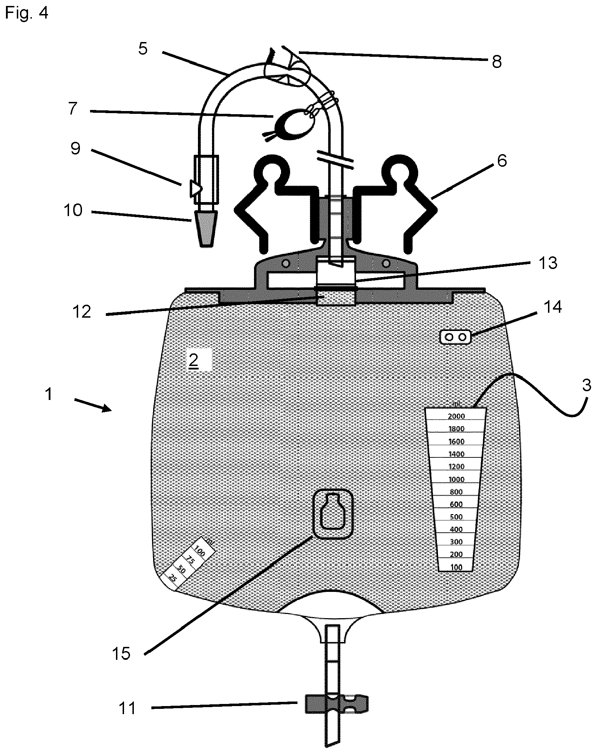

Figur 4 zeigt die Rückseite eines erfindungsgemäßen Urinbeutels 1. Man erkennt hier den Ablasshahn 11 und den Halter 15 für diesen. Solange der Ablasshahn 11 in den Halter 15 eingehängt ist, sieht man ihn von vorne nicht, wie es die Figuren 2 und 3 zeigen. In Figur 4 ist eine deckend bedruckte Rückfolie zu sehen, bei der Ausnehmungen im Druck für zwei Skalierungen 3, 3' vorgesehen sind. Außerdem ist eine sichelförmige Ausnehmung 17 im Druck vorhanden, durch die man die Qualität des Urins beurteilen kann.

### Bezugszeichenliste

- 1: Urinbeutel
- 2: Flüssigkeitssammelbehälter
- 3: Skalierung
- 4': separate Folie
- 4: deckender Druck
- 5: Schlauch
- 6: Aufhängung
- 7: Schlauchfixierklemme
- 8: Ritsch-Ratsch-Klemme
- 9: Probenentnahmestelle
- 10: Schutzkappe
- 11: Ablasshahn
- 12: Tropfkammer
- 13: Antirefluxventil
- 14: Belüftung
- 15: Halter für Ablasshahn
- 16: Fenster durch Ausnehmung im Druck
- 17: sichelförmige Ausnehmung im Druck

## Patentansprüche

1. Hygienebeutel (1) zur Aufnahme von Körperflüssigkeit, umfassend einen Flüssigkeitssammelbehälter (2), der durch eine vorderseitige Folie und eine rückseitige Folie, die miteinander verbunden sind, gebildet wird, **dadurch gekennzeichnet, dass** die vorderseitige Folie einen deckenden Druck (4) aufweist, durch den die Körperflüssigkeit nicht sichtbar ist.

2. Hygienebeutel (1) gemäß Anspruch 1, **dadurch gekennzeichnet, dass** der Druck (4) Ausnehmungen aufweist, die ein Fenster (16) bilden, durch das der Füllstand und/oder die Qualität der Körperflüssigkeit beurteilt werden kann.

3. Hygienebeutel (1) gemäß Anspruch 2, **dadurch gekennzeichnet, dass** die das Fenster (16) bildende Ausnehmung im Druck (4) rund, insbesondere kreisförmig oder oval, oder eckig, insbesondere rautenförmig oder quadratisch, ist, wobei vorzugsweise mehrere Ausnehmungen in einer Linie übereinander vorgesehen sind.

4. Hygienebeutel (1) gemäß Anspruch 2, **dadurch gekennzeichnet, dass** die Ausnehmung im Druck (4) streifenförmig ist und ein Fenster (16) in Form der Kontur eines Gegenstandes, beispielsweise eines Blattes oder einer Blume, bildet.

5. Hygienebeutel (1) gemäß einem der Ansprüche 1 bis 4, **dadurch gekennzeichnet, dass** die rückseitige Folie eine durchsichtige Folie mit einem weiteren Druck ist.

6. Hygienebeutel (1) gemäß Anspruch 5, **dadurch gekennzeichnet, dass** der weitere Druck eine Skalierung ist.

7. Hygienebeutel (1) gemäß Anspruch 5, **dadurch gekennzeichnet, dass** der weitere Druck deckend ist, so dass die rückseitige Folie undurchsichtig ist.

8. Hygienebeutel (1) gemäß Anspruch 7, **dadurch gekennzeichnet, dass** der weitere Druck eine Ausnehmung aufweist, durch welche eine Skalierung (3, 3') sichtbar ist.

9. Hygienebeutel (1) gemäß Anspruch 7, **dadurch gekennzeichnet, dass** der weitere Druck eine, vorzugsweise sichelförmige, Ausnehmung (17), insbesondere am unteren Rand der rückseitigen Folie, aufweist.

10. Hygienebeutel (1) gemäß einem der Ansprüche 1 bis 9, **dadurch gekennzeichnet, dass** ein Ablassschlauch und/oder ein Einlaufschlauch auf der Rückseite des Hygienebeutels angebracht ist/sind.
